# FASCICULE DE BREVET EUROPEEN

(11) **EP 2 739 260 B1**
(45) Date de publication et mention de la délivrance du brevet: **27.09.2017**
(21) Numéro de dépôt: 12750477.7
(22) Date de dépôt: 03.08.2012
(51) Int. Cl.: A61K 8/42, A61Q 5/10, A61Q 19/04, A61K 31/164, A61P 17/00

(54) **UTILISATION DE COMPOSÉS CANNABINOÏDES POUR STIMULER LA MÉLANOGÉNÈSE**
VERWENDUNG VON CANNABINOIDVERBINDUNGEN ZUR FÖRDERUNG VON MELANOGENESE
USE OF CANNABINOID COMPOUNDS FOR STIMULATING MELANOGENESIS

(30) Priorité: 05.08.2011 FR 1157213
(43) Date de publication de la demande: 11.06.2014
(73) Titulaire: L'OREAL, 75008 Paris (FR)
(72) Inventeur: ZUCCOLO, Michela, F-75011 Paris (FR); JOURDAIN, Roland, F-92360 Meudon La Forêt (FR); BRETON, Lionel, F-78000 Versailles (FR); MACCARRONE, Mauro, I-67100 L'aquila (IT)
(74) Mandataire: Pillet, Anne-Helene
(86) Numéro de dépôt international: PCT/FR2012/051846
(87) Numéro de publication internationale: WO 2013/021128

(56) Documents cités:
- EP-A2- 0 355 842
- WO-A1-95/11003
- WO-A1-2009/071422
- WO-A2-2006/024958
- WO-A2-2009/158499
- FR-A1- 2 848 106
- SOFIA MAGINA ET AL: "Inhibition of basal and ultraviolet B-induced melanogenesis by cannabinoid CB1 receptors: a keratinocyte-dependent effect", ARCHIVES OF DERMATOLOGICAL RESEARCH, vol. 303, no. 3, 5 février 2011 (2011-02-05), pages 201-210, XP055026334, ISSN: 0340-3696, DOI: 10.1007/s00403-011-1126-z cité dans la demande

## Description

La présente invention concerne l'utilisation cosmétique et/ou dermatologique de composés cannabinoïdes, dans une composition pour colorer et/ou pigmenter la peau et/ou les poils et/ou les cheveux.

La couleur des cheveux et de la peau humaine est fonction de différents facteurs et notamment des saisons de l'année, de l'origine ethnique, du sexe et de l'âge. Elle est principalement déterminée par la concentration, dans les kératinocytes, de la mélanine produite par les mélanocytes. Les mélanocytes sont les cellules spécialisées qui, par l'intermédiaire d'organelles particuliers, les mélanosomes, synthétisent la mélanine. Cette synthèse de mélanine, ou mélanogenèse, est le résultat de l'interaction entre le α-MSH (α-melanocyte stimulating hormone) et son récepteur MC1 R (récepteur de la mélanocortine de type 1), interaction qui active une cascade de signalisation induisant l'enzyme tyrosinase *via* le facteur de transcription MITF.

La mélanogénèse est particulièrement complexe et fait intervenir schématiquement les principales étapes suivantes :
Tyrosine ---> Dopa ---> Dopaquinone ---> Dopachrome ---> Mélanine

La tyrosinase (monophénol dihydroxyl phénylalanine : oxygen oxydoreductase / EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en Dopaquinone.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. Ils naissent du réticulum endoplasmique sous formes de vacuoles sphériques appelées prémélanosomes. Les prémélanosomes contiennent un substrat protéinique amorphe, mais pas d'enzymes mélanogènes. Au cours de la maturation du prémélanosome, le substrat amorphe s'organise en une structure fibrillaire orientée dans l'axe longitudinal du mélanosome. On distingue quatre stades du développement du mélanosome correspondant à l'intensité de la mélanisation.

La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A. Ces rayonnements provoquent le brunissement de la peau mais en contrepartie ils sont susceptibles d'induire une altération de celle-ci, notamment dans le cas d'une peau sensible ou d'une exposition continuelle au rayonnement solaire. La peau vieillit prématurément, devient sèche et se caractérise par de nombreuses rides et taches de sénescence. Il est donc souhaitable de trouver une alternative au bronzage naturel qui soit compatible avec les exigences de telles peaux.

La recherche de composés pigmentants trouve également un intérêt dans le traitement des maladies de la pigmentation comme par exemple le vitiligo qui est une maladie auto-immune se caractérisant par l'apparition sur la peau de plaques blanches liées à un défaut de pigmentation ou encore le pityriasis versicolor dû à la levure *Malassezia furfur* (taches pouvant être achromiantes d'emblée ou après exposition au soleil).

Un autre défaut de pigmentation est l'apparition des cheveux blancs chez l'homme (canitie ou blanchissement naturel des cheveux) qui peut être soit une manifestation visible du processus de vieillissement (canitie sénile), soit lié à une prédisposition génétique. La pigmentation du cheveu et des poils requiert la présence de mélanocytes au niveau du bulbe du follicule pileux. Il est maintenant admis que la canitie est associée à une diminution de la quantité de mélanine dans la tige pilaire. Le maintien d'une coloration constante de la chevelure étant une aspiration importante, il est donc souhaitable de pouvoir lutter contre l'apparition de ces signes visibles du vieillissement, c'est à dire de maintenir ou rétablir la coloration des poils et/ou des cheveux.

Il serait ainsi particulièrement intéressant de disposer de nouveaux moyens pour faciliter et/ou améliorer la pigmentation de la peau et/ou des poils et/ou des cheveux dans le domaine de la dermatologie et de la cosmétique. Ces nouveaux moyens seraient notamment utiles pour prévenir et/ou limiter et/ou stopper le développement de la canitie et même maintenir la pigmentation naturelle des cheveux et/ou des poils gris ou blancs.

Il existe donc un réel besoin de produit facilitant et/ou améliorant la pigmentation de la peau et/ou des poils et/ou des cheveux.

De nombreuses solutions ont été proposées dans le domaine de la coloration artificielle.

Dans le domaine des colorants exogènes, la dihydroxyacétone, ou DHA, est un produit particulièrement intéressant qui est couramment utilisé en cosmétique comme agent de bronzage artificiel de la peau. Appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

L'érythrulose est également une substance utilisée dans le même but. Ces composés agissent en se fixant sur les acides aminés, peptides et protéines du *stratum corneum,* selon la réaction de Maillard (réaction non enzymatique entre un sucre et une amine).

Ces colorations obtenues avec des composés de type autobronzant tels que définis précédemment apparaissent au bout de quelques heures, mais disparaissent assez rapidement, et ne sont pas toujours réparties de façon homogène sur la peau.

Afin d'obtenir des colorations qui durent dans le temps, il a été proposé dans l'art antérieur d'agir directement sur le processus biologique de la mélanogénèse par l'intermédiaire de l'α-MSH ou des prostaglandines, de façon à stimuler la biosynthèse de la mélanine, avec ou sans action des UV. Par exemple, il a été proposé dans les documents WO 95/17161, WO 95/11003, WO 95/01773, WO 94/04674, WO 94/04122, EP 0 585 018, WO 93/10804, WO 92/20322 ou WO 91/07945, des solutions aussi variées que des compositions contenant un inhibiteur de phosphodiestérases, de prostaglandines, des fragments d'ADN ou encore des dérivés de la tyrosine.

Alors que l'on pensait qu'il n'existait aucune autre voie de mélanogénèse qui ne dépende pas de α-MSH et de MC1R, la Demanderesse a découvert, de façon surprenante, que certains composés cannabinoïdes permettent d'induire la biosynthèse de la mélanine, et d'obtenir une coloration de la peau plus homogène et durable que lors de l'utilisation de colorants exogènes. La nouvelle voie décrite se traduit par une pigmentation plus rapide. Par ailleurs, cette pigmentation naturelle est physiologiquement plus efficace qu'un pigment synthétique.

Par « composé cannabinoïde », on entend, au sens de la présente invention, toute substance, composé simple ou complexe, d'origine naturelle ou synthétique, qui active les récepteurs cannabinoïdes présents dans le corps et notamment dans la peau. Il en existe trois grandes catégories : les cannabinoïdes végétaux ou phytocannabinoïdes, les cannabinoïdes endogènes ou endocannabinoïdes et les cannabinoïdes synthétiques, élaborés en laboratoires.

Les endocannabinoïdes sont des lipides actifs qui agissent comme ligands des récepteurs CB1 et CB2. Ces récepteurs appartiennent à la superfamille des récepteurs transmembranaires, couplés aux protéines G. Le récepteur CB1 est codé par le gène CNR1 présent sur le chromosome 6. L'un des principaux endocannabinoïdes est l'anandamide ou AEA (*N*-arachidonoylethanolamine). La synthèse de l'AEA dépend de l'enzyme *N*-acyl-phosphatidylethanolamines (NAPE)-specific phospholipase D (NAPE-PLD), alors que sa dégradation a lieu par hydrolyse par l'enzyme FAAH (fatty acid amide hydrolase). Le méthanandamide ou mAEA (R(+)-arachidonyl-1'-hydroxy-2'-propylamide) est un analogue non hydrolysable de l'AEA et est, comme l'AEA, un ligand agoniste des récepteurs CB1, CB2 et TRPV1. L'arachidonoyl 2'-chloroethylamide ou ACEA est aussi un analogue de l'AEA et est également un ligand agoniste du récepteur CB1. L'entrée des endocannabinoïdes dans la cellule est saturable et énergie-dépendante, et elle a lieu *via* le transporteur membranaire d'endocannabinoïdes (EMT).

L'AEA et les protéines qui se lient à ce composé, le transportent, le synthétisent et l'hydrolysent font partie du système endocannabinoïde (ECS). Les endocannabinoïdes ont été identifiés - à la fois chez l'homme et chez la souris - dans un large nombre de types cellulaires de la peau et des structures associées, tels les kératinocytes épidermiques, les mastocytes du derme, les sébocytes et l'épithélium du follicule pileux (Maccarrone et al, J Biol Chem, 2003, Dobrosi et al., FASEB J, 2008). Il a été montré que les éléments du ECS contrôlent la prolifération, la différentiation, l'apoptose et la production de cytokines des cellules de la peau, ce qui suggère un rôle de ce système dans l'homéostasie de l'épiderme. Par ailleurs, la liaison de ligands agonistes comme l'AEA sur les récepteurs cannabinoïdes dans la peau entraine une atténuation des processus inflammatoires, tandis que des antagonistes aggravent des épisodes inflammatoires cutanés comme dans les pathologies de type dermatites allergiques.

L'anandamide a déjà été décrit pour ses effets anti-bactérien (WO 2009/158499), anti-douleur ou encore anti-inflammatoire notamment via les voies de signalisation des récepteurs vanilloides. Il n'a cependant jamais été décrit comme agissant sur des mélanocytes ou comme pouvant avoir un effet sur la production de mélanine via des récepteurs cannabinoïdes comme dans la présente invention.

Magina et al. (Arch Dermatol Res. 2011) décrit une inhibition de la mélanogénèse suite à l'exposition de co-cultures de kératinocytes et de mélanocytes à un rayonnement UV. Les mélanocytes utilisés dans ces co-cultures sont particuliers puisque ce sont ici des cellules de mélanome humain, c'est-à-dire des cellules cancéreuses. Les auteurs émettent l'hypothèse que cet effet sur la mélanogénèse serait dû à l'action de médiateurs endocannabinoïdes secrétés par les kératinocytes et agissant sur les mélanocytes environnants. La Demanderesse est donc allée à l'opposé de ce qui est suggéré dans cette publication en testant des ligands des récepteurs cannabinoïdes, pour leur effet stimulateur de la mélanogénèse.

Ainsi, il est décrit l'utilisation cosmétique (non-thérapeutique) d'au moins un activateur du récepteur CB1, préférentiellement au moins un activateur du récepteur CB1, à l'exception du 2-arachidonoylglycerol (2-AG) seul (i.e. quand le 2-AG n'est pas associé à un autre activateur du récepteur CB1, comme agent de coloration des matières kératiniques.

Un activateur du récepteur CB1 est toute substance, composé simple ou complexe, d'origine naturelle ou synthétique, qui se fixe au récepteur CB1 et induit une réponse biologique similaire à celle que l'on obtient avec le ligand naturel de ce récepteur qui active cette réponse ou qui augmente la libération et/ou la synthèse des endocannabinoïdes. Cette caractéristique de ces composés peut être facilement vérifiée par les méthodes connues de l'homme du métier, à savoir respectivement par une méthode de mesure d'affinité réceptorielle sélective (dite de « binding ») couplée à une mesure de l'activité fonctionnelle dudit composé ou de ladite substance (voir notamment J. Med. Chem. 2007, 50, 3851-3856) ou par une méthode de libération contrôlée des endocannabinoïdes (dites méthodes de « release »).

L'activateur du récepteur CB1 peut être choisi préférentiellement parmi les ligands agonistes du récepteur CB1 ou les inducteurs de libération d'endocannabinoïdes (autrement appelés inducteurs de « release ») et plus préférentiellement parmi les ligands agonistes du récepteur CB1 et encore plus préférentiellement parmi les ligands agonistes du récepteur CB1 à l'exception du 2-arachidonoylglycerol (2-AG) seul (i.e. quand le 2-AG n'est pas associé à un autre ligand agoniste du récepteur CB1).

Par « inducteurs de libération des endocannabinoïdes » on entend désigner, au sens de la présente invention, toute substance, composé simple ou complexe, d'origine naturelle ou synthétique induisant la synthèse et/ou libération endogène d'endocannabinoïdes. Ils sont aussi appelés agonistes fonctionnels.

Un ligand agoniste du récepteur CB1 est, selon la présente invention, toute substance, composé simple ou complexe, d'origine naturelle ou synthétique qui va se fixer sur un récepteur cannabinoïde présent dans le corps humain, en particulier sur le récepteur CB1 et qui va induire une réponse biologique similaire à celle que l'on obtient avec le ligand naturel de ce récepteur qui active cette réponse. Les ligands agonistes du récepteur CB1 sont préférentiellement choisis parmi les composés cannabinoides, l'anandamide et ses analogues, notamment le méthanandamide, l'arachinoyl 2'-chloroéthylamide, le linoleyl ethanolamide, l'éther de noladine, le CP 55,940 ([(-)-cis-3-[2-Hydroxy-4-(1,1-dimethylheptyl)phenyl]-trans-4-(3-hydroxypropyl) cyclohexanol], le O-2050 [(6aR, 10aR)-3-(1-Methanesulfonylamino-4-hexyn-6-yl)-6a, 7,10,10a-tetrahydro-6,6,9-trimethyl-6H-dibenzo[b,d]pyrane] et leurs mélanges.

Ces agonistes sont capables de brunir ou d'assombrir les matières kératiniques et/ou d'améliorer et/ou d'accélérer et/ou de restaurer la coloration ou la pigmentation des matières kératiniques et notamment de la peau et/ou des cheveux.

La présente invention décrit l'utilisation cosmétique non-thérapeutique d'au moins un composé cannabinoïde choisi parmi les composés répondant à la formule générale (I) : 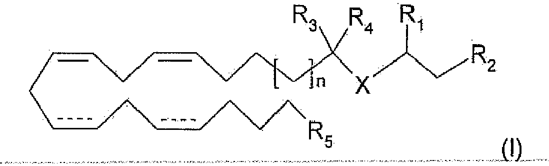 ainsi que ses isomères géométriques (cis/trans ou Z/E) et optiques (énantiomères), ses sels d'acide ou de base cosmétiquement acceptables, et ses hydrates tels que les solvates ; composés de formule **(I)** dans laquelle :
- **R₁** représente un atome d'hydrogène ou un groupement alkyle en C₁-C₃₀, préférentiellement un groupement alkyle en C₁-C₆, encore plus préférentiellement un groupement méthyle ; ledit groupement alkyle pouvant éventuellement être substitué par un groupement hydroxyle (OH),
- **R₂** représente un atome d'halogène ou un groupement choisi parmi hydroxyle, thiol (SH), et amino éventuellement substitué par un ou deux groupement alkyle en C₁-C₆, plus particulièrement choisi parmi un halogène tel que le chlore et un groupement hydroxyle ;
- **R₃** et **R₄** forment ensemble avec l'atome de carbone qui les porte un groupement oxo ou alors R₃ et R₄ représentent un atome d'hydrogène ;
- **R₅** représente un atome d'hydrogène ou un groupement alkyle en C₁-C₆ tel que méthyle ou éthyle ;
- **X** représente un hétéroatome choisi parmi les atomes d'oxygène, de soufre et le groupement divalent -N(R₆)-, avec R₆ représentant un atome d'hydrogène ou un groupement alkyle en C₁-C₆;
- 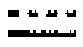 représente une simple ou une double liaison ;
- n vaut 1, 2 ou 3, préférentiellement 1 ou 2 ;
étant entendu que lorsque **R₃** et **R₄** forment ensemble un groupement oxo avec l'atome de carbone qui les porte et que **X** représente un atome d'oxygène, alors **R₁** ne peut pas représenter un groupement hydroxyméthyle.
comme agent de coloration des matières kératiniques.

Par « matière kératinique », on entend la peau (du visage, du corps, du cuir chevelu), les cheveux, les cils, les sourcils et les ongles. Préférentiellement, on entend par « matière kératinique », la peau et/ou les cheveux.

Par « agent de coloration », on entend toute substance ou composé susceptible de brunir ou d'assombrir les matières kératiniques et/ou d'améliorer et/ou de favoriser et/ou d'accélérer et/ou de restaurer la coloration ou la pigmentation des matières kératiniques.

Ce composé cannabinoïde est selon l'invention choisi parmi l'anandamide (AEA) et ses analogues. Par composés « analogues », on entend des composés d'origine synthétique ou naturelle, de structure chimique analogue à l'anandamide et qui sont capables de se fixer sur les mêmes récepteurs. Selon l'invention, les analogues de l'anandamide sont choisis parmi le methanandamide (mAEA), l'arachidonoyl 2'-chloroetylamide (ACEA), le linoleyl ethanolamide, le docosatetraenyl ethanolamide et le 2-arachidonyl glyceryl ether (2-AGE, Noladin ether).

L'AEA, le mAEA et l'ACEA, le linoleyl ethanolamide, le docosatetraenyl ethanolamide et le 2-arachidonyl glyceryl ether, de structure chimique proche, ont les formules chimiques suivantes (l'abréviation =O signifie groupement oxo, dl signifie « double liaison » et sl signifie « simple liaison ») :

| | **R₁** | **R₂** | **R₃ et R₄** | **R₅** | **X** | **R₆** | ---- | **n** |
|---|---|---|---|---|---|---|---|---|
| Anandamide | H | OH | =O | C₂H₅ | N(R₆) | H | dl | 1 |
| 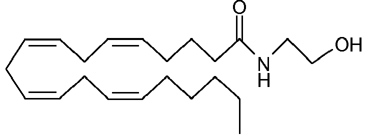 | | | | | | | | |
| Methanandamide | CH₃ | OH | =O | C₂H₅ | N(R₆) | H | dl | 1 |
| 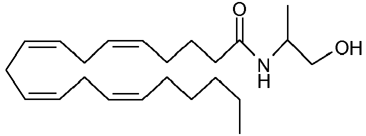 | | | | | | | | |
| Arachidonoyl 2'-chloroethylamide | H | Cl | =O | C₂H₅ | N(R₆) | H | dl | 1 |
| 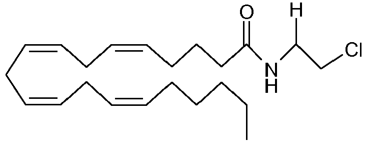 | | | | | | | | |
| Lioneyl ethanolamide | CH₃ | OH | =O | H | N(R₆) | H | sl | 1 |
| 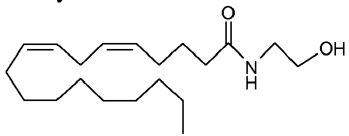 | | | | | | | | |
| Docosatetraenyl ethanolamide | H | OH | =O | C₂H₅ | N(R₆) | H | dl | 2 |
| 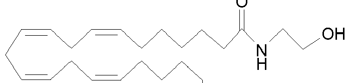 | | | | | | | | |
| 2-arachidonyl glyceryl ether | CH₃ substitué par OH | OH | =O | C₂H₅ | O | H | dl | 1 |
| 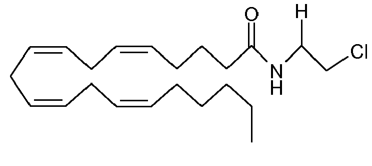 | | | | | | | | |

Le brunissement ou l'assombrissement de la peau ou l'amélioration, l'accélération, la restauration de la coloration ou de la pigmentation peuvent être appréciés visuellement ou par des méthodes de mesure physique de la coloration de la peau ou des cheveux, bien connues de l'homme du métier (notamment par le système de mesure colorimétrique (L*, a*, b*)).

Le ligand agoniste du récepteur CB1 ou le composé cannabinoïde tel que défini ci-dessus, peut être présent dans une composition à une concentration comprise entre 0,00001% à 10% en poids, de préférence entre 0,001% à 5% en poids, notamment entre 0,01 à 1% en poids, par rapport au poids total de la composition.

Préférentiellement, l'activateur du récepteur CB1 ou le composé cannabinoïde, tel que défini ci-dessus, est utilisé comme agent pour augmenter la pigmentation des matières kératiniques et/ou pour stimuler la mélanogénèse, notamment par l'intermédiaire d'une activation des récepteurs CB1 et/ou comme agent de brunissement de la peau et/ou comme agent de bronzage artificiel de la peau.

Par cette utilisation, la peau apparaitra plus sombre, plus brune, plus bronzée et/ou plus rapidement bronzée.

Par « bronzage artificiel », on entend un brunissement, un assombrissement ou une augmentation de la pigmentation de la peau non directement liée à une exposition au soleil ou aux UVA et/ou UVB. Le brunissement obtenu sera proche d'un bronzage naturel.

Par « bronzage naturel », on entend un brunissement, un assombrissement ou une augmentation de la pigmentation de la peau directement liée à une exposition au soleil ou aux UVA et/ou UVB.

Plus particulièrement, l'activateur du récepteur CB1 ou le composé cannabinoïde, tel que défini ci-dessus est utilisé selon l'invention comme agent pour ralentir ou traiter la canitie ou le blanchissement naturel des cheveux. Par l'utilisation d'un activateur du récepteur CB1 ou de composé cannabinoïde selon l'invention, on peut notamment maintenir et/ou rétablir la pigmentation naturelle des cheveux et/ou des poils.
Par le terme « traiter », on entend aussi bien prévenir, diminuer ou ralentir. Il est décrit aussi un composé cannabinoïde ou un activateur du récepteur CB1, tel que défini ci-dessus, pour son utilisation dans le traitement d'affection dermatologique à composante dyschromique telle que le vitiligo ou le pityriasis versicolor. Il est décrit aussi l'utilisation d'au moins un activateur du récepteur CB1 ou un composé cannabinoïde tel que défini ci-dessus, pour la préparation d'une composition destinée à traiter toute affection dermatologique à composante dyschromique telle que le vitiligo ou le pityriasis versicolor.

Par « affection dermatologique à composante dyschromique », on entend toute affection, maladie ou pathologie dans laquelle on observe une pigmentation anormale (préférentiellement une pigmentation insuffisante) sur une partie ou sur la totalité de la peau d'un individu.

La composition pour l'utilisation est en particulier adaptée à une application topique ou adaptée à une administration orale et contient par ailleurs un milieu physiologiquement acceptable. Les activateurs du récepteur CB1 ou les composés cannabinoïdes, seuls ou en mélange, ainsi que la composition les comprenant, peuvent être utilisés en application topique sur les matières kératiniques ou en administration orale. La composition peut être notamment une composition cosmétique ou une composition dermatologique. Par « adaptée à une application topique » ou « adaptée à une administration orale), on entend respectivement « convenant à une application topique » ou « convenant à une administration orale ».

Par « milieu physiologiquement acceptable », on entend, selon l'invention, un milieu cosmétiquement ou pharmaceutiquement acceptable compatible avec la peau (y compris le cuir chevelu), les cheveux, les muqueuses et/ou les yeux. Par « milieu cosmétiquement acceptable », on entend un milieu sans odeur ou aspect désagréable, et qui ne génère pas de picotement, de tiraillement ou de rougeur inacceptable pour l'utilisateur, lors de son application topique sur la peau ou ses annexes.

Le milieu physiologiquement acceptable sera adapté à la nature du support sur lequel doit être appliqué la composition ainsi qu'à la forme sous laquelle la composition est destinée à être conditionnée, notamment solide ou fluide à température ambiante et pression atmosphérique.

La composition peut également comprendre tous les additifs cosmétiques usuels tels que l'eau, les solvants, les huiles, les cires, les pigments, les charges, les tensioactifs, les actifs cosmétiques ou dermatologiques, les filtres UV, les polymères, les gélifiants, les conservateurs et les parfums.

Bien entendu, l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition peut se présenter sous toutes les formes galéniques normalement utilisées dans les domaines cosmétiques et dermatologiques; elle peut être notamment sous forme d'une solution aqueuse, hydroalcoolique ou huileuse, éventuellement gélifiée, d'une dispersion du type lotion éventuellement biphasée, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Un autre objet de l'invention est un procédé cosmétique non-thérapeutique de coloration ou de pigmentation de la peau comprenant l'application topique sur la peau ou l'administration orale, d'une composition comprenant, dans un milieu physiologiquement acceptable, un composé cannabinoïde ou un activateur du récepteur CB1, tel que défini selon l'invention ci-dessus.

Préférentiellement, ce procédé cosmétique est un procédé de brunissement de la peau ou un procédé de bronzage artificiel de la peau.

L'invention concerne également un procédé cosmétique non-thérapeutique de traitement de la canitie comprenant l'application topique sur les cheveux ou l'administration orale, d'une composition comprenant, dans un milieu physiologiquement acceptable, un composé cannabinoïde ou un activateur du récepteur CB1, tel que défini selon l'invention ci-dessus.

Dans l'un ou l'autre de ces procédés, l'activateur du récepteur CB1 ou le composé cannabinoïde peut être présent à une concentration comprise entre à 0,00001 et 10% en poids par rapport au poids total de la composition, de préférence entre 0,001% à 5% en poids, notamment entre 0,01 à 1% en poids.

L'un ou l'autre de ces procédés cosmétiques peut comprendre une application ou une administration unique. Selon un mode de réalisation particulier, l'application ou l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Il est également décrit un procédé de sélection d'un actif favorisant la pigmentation des matières kératiniques comprenant :
a) une étape de pré-sélection d'un activateur du récepteur CB1,
b) une étape de sélection finale d'un activateur du récepteur CB1 présélectionné à l'étape précédente, en tant qu'actif favorisant la pigmentation des matières kératiniques, lorsque la quantité de mélanine produite par des mélanocytes mis en contact avec ledit activateur est supérieure à celle produite par des mélanocytes n'ayant pas été en contact avec cet activateur du récepteur CB1.

L'activateur du récepteur CB1 est tel que défini ci-dessus. Il peut être un ligand agoniste du récepteur CB1 ou un inducteur de libération d'endocannabinoïdes, le ligand agoniste du récepteur CB1 étant préférentiellement choisi parmi les composés cannabinoïdes répondant à la formule (I), tels que définis ci-dessus.

Le caractère activateur ou agoniste du récepteur CB1, d'un composé ou d'une substance est évalué par toute méthode connue de l'homme du métier, notamment par une méthode de mesure d'affinité réceptorielle sélective (dite de « binding ») couplée à une mesure de l'activité fonctionnelle dudit composé ou de ladite substance (voir notamment J. Med. Chem. 2007, 50, 3851-3856) ou par une méthode de libération contrôlée des endocannabinoïdes (dite méthode de « release »).

La mesure d'affinité réceptorielle sélective peut être réalisée en mettant en contact des récepteurs CB1 (ou des membranes de cellules comprenant ces récepteurs) avec des ligands marqués (e.g. des ligands radiomarqués, immunofluorescents...), en éliminant les ligands marqués qui ne se sont pas liés aux récepteurs et en déterminant la quantité de ligands marqués qui se sont liés aux récepteurs.

La mesure de l'activité fonctionnelle d'un composé ou d'une substance pour valider son caractère agoniste du récepteur CB1 peut être réalisée en mesurant la quantité d'AMP cyclique (AMPc) produite par une culture cellulaire (i.e. une culture de mélanocytes ou une coculture de mélanocytes et de kératinocytes ou un épiderme reconstruit pigmenté ou un explant de peau), suite à l'ajout de forskoline et dudit composé ou de ladite substance. Si une diminution de la quantité d'AMPc est observée suite à l'ajout du composé ou de la substance alors son caractère activateur ou agoniste du récepteur CB1 est validé. L'AMP cyclique est en effet l'un des composés connus dont la synthèse est inhibée suite à l'activation du récepteur CB1. La forskoline est connue comme augmentant la synthèse d'AMPc.

Une méthode de libération contrôlée des endocannabinoides se fera par mise en contact d'un composé ou d'une substance avec une culture cellulaire (i.e. une culture de mélanocytes ou une coculture de mélanocytes et de kératinocytes ou un épiderme reconstruit pigmenté ou un explant de peau) et par mesure de la quantité d'endocannabinoides produite.

Dans l'étape b) la quantité de mélanine produite par des mélanocytes peut être mesurée sur une culture de mélanocytes ou sur une coculture de mélanocytes et de kératinocytes ou sur un épiderme reconstruit pigmenté ou sur un explant de peau. A l'étape b), l'activateur du récepteur CB1 présélectionné à l'étape a) peut être ajouté à une concentration déterminée sur l'une ou l'autre de ces cultures de cellules et la quantité de mélanine produite par les mélanocytes présents dans ces cultures est mesurée. Une mesure contrôle est effectuée sur l'une ou l'autre de ces cultures de cellules sans avoir ajouté d'activateur du récepteur CB1.

La sélection finale d'un activateur du récepteur CB1 en tant qu'actif favorisant la pigmentation des matières kératiniques à l'étape b) du procédé de sélection est faite par comparaison de la quantité de mélanine produite par des mélanocytes mis en contact avec ledit activateur du récepteur CB1 et de la quantité de mélanine produite par des mélanocytes n'ayant pas été mis en contact avec ledit activateur du récepteur CB1. Un activateur du récepteur CB1 peut être ainsi sélectionné en tant qu'actif favorisant la pigmentation des matières kératiniques, s'il augmente d'au moins 10%, préférentiellement d'au moins 20%, encore plus préférentiellement d'au moins 30%, la quantité de mélanine produite par des mélanocytes n'ayant pas été en contact avec ledit activateur du récepteur CB1.

Les conditions de culture des mélanocytes seuls ou en coculture avec des kératinocytes ainsi que les méthodes de préparation d'épiderme reconstruit pigmenté (voir notamment EP 1 878 790) ou d'expiant de peau sont connues de l'homme du métier.

La mesure de la quantité de mélanine produite par les mélanocytes, éventuellement en co-culture avec des kératinocytes ou présents dans l'épiderme reconstruit pigmenté ou dans un explant de peau est faite selon des méthodes connues de l'homme du métier (voir par exemple Xiao et al., Arch Dermatol Res 2007 ; 299 : 245-57). Préférentiellement, cette mesure est réalisée par spectrophotométrie et encore plus préférentiellement par spectrophotométrie à 450 nm.

La présente invention décrit également un procédé de sélection d'agent de coloration ou d'actif favorisant la pigmentation des matières kératiniques comprenant :
a) une étape de mise en contact d'un produit à tester (P) avec des mélanocytes,
b) une étape de mesure de la quantité de mélanine produite par les mélanocytes,
c) une étape de comparaison de la quantité de mélanine mesurée à l'étape b) à la quantité de mélanine (i) produite par des mélanocytes ayant été mis en contact avec le produit (P) et un composé inhibiteur de l'activité du récepteur CB1,
d) une étape de comparaison de la quantité de mélanine mesurée à l'étape b) à la quantité de mélanine (ii) produite par des mélanocytes n'ayant été en contact ni avec le produit (P), ni avec un composé inhibiteur de l'activité du récepteur CB1,
e) une étape de sélection d'un produit (P) en tant qu'agent de coloration ou actif favorisant la pigmentation des matières kératiniques, lorsque la quantité de mélanine mesurée à l'étape b) est significativement plus importante que la quantité de mélanine ii) et que la quantité de mélanine (i) n'est pas significativement différente de la quantité de mélanine (ii),
les conditions de mesure de la quantité de mélanine étant les mêmes dans les étapes a, b, c et d.

La mise en contact du produit à tester (P) éventuellement associé à un composé inhibiteur de l'activité du récepteur CB1 avec des mélanocytes est faite préférentiellement par mise en contact de ces composés avec une culture de mélanocytes, une coculture de mélanocytes et de kératinocytes, un épiderme reconstruit pigmenté ou un explant de peau.

Par « composé inhibiteur de l'activité du récepteur CB1 », on entend, au sens de l'invention, toute substance, composé simple ou complexe,d'origine naturelle ou 40 synthétique, capable d'inhiber ou de diminuer l'activité du récepteur CB1, donc qui l'empêchera de produire ses effets. Ce terme peut donc désigner par exemple des enzymes de dégradation de l'anandamide (s'il n'y a plus de ligand disponible, il n'y a plus d'activation). Par ce terme, on désigne donc aussi toute substance ou moyen moléculaire (ARN interférent, siRNA, miRNA) susceptible d'inhiber spécifiquement l'expression du récepteur elle-même.

Un homme du métier identifiera très facilement le caractère inhibiteur d'une substance ou d'un composé par les méthodes de mesure de l'activité d'un récepteur, connues de l'état de la technique. Un exemple d'une telle méthode est donné dans le document Endocrinology 2009;150:4692-4700.

Comme composé inhibiteur de l'activité du récepteur CB1, on peut citer le *N-*Piperidino-5-(4-chlorophenyl)-1-(2,4-dichloro-phenyl)-4-methyl-3-pyrazole carboxamide), également connu sous les noms de SR1, de SR141716 ou de rimonabant et notamment commercialisé par la société Sigma Aldrich ou sous la marque Acomplia™ ou Zimulti™ par la société Sanofi Aventis.

La quantité de composé inhibiteur de l'activité du récepteur CB1 pouvant être utilisée est déterminée de telle façon que l'activité du récepteur CB1 est réduite de 80%, préférentiellement 90%, encore plus préférentiellement 100%. A titre d'exemple, on peut utiliser 0,1 µM de SR1 pour inhiber totalement l'activité du récepteur CB1.

L'un ou l'autre de ces procédés de sélection, permet de sélectionner spécifiquement des produits qui peuvent être utilisés comme agents pour favoriser la coloration de la peau et/ou des poils et/ou des cheveux. Ces produits ont la particularité de stimuler la mélanogénèse ou la pigmentation de la peau et/ou des poils et/ou des cheveux, par l'intermédiaire de l'activation du récepteur CB1.

Dans la description et dans les exemples suivants, sauf indication contraire, les pourcentages sont des pourcentages en poids et les plages de valeurs libellées sous la forme "entre ... et ..." incluent les bornes inférieure et supérieure précisées. Les ingrédients sont mélangés, avant leur mise en forme, dans l'ordre et dans des conditions facilement déterminées par l'homme de l'art.

### Exemple 1 : Mise en évidence de l'effet pro-pigmentant de 3 composés cannabinoïdes

### 1. Matériels et méthodes

### 1.1. Composés testés

Anandamide (AEA) et methanandamide (mAEA) sont fournis par Sigma Chemical Co. (St. Louis, MO). L'Arachidonoyl 2'-chloroethylamide (ACEA) est fourni par Cayman Chemical (Ann Arbor, MI).

### 1.2. Réactifs utilisés

Les composés SR1 = *N*-Piperidino-5-(4-chlorophenyl)-1-(2,4-dichloro-phenyl)-4-methyl-3-pyrazole carboxamide (antagoniste spécifique du récepteur CB1) et SR2 = *N*-[(1*S*)-endo-1,3,3-trimethy-1-bicyclo [2.2.1]-heptan-2-yl]5-(4-choro-3-methyl-phenyl)-1-(4-methyl-benzyl)-pyrazole-3-carboxamide (antagoniste spécifique du récepteur CB2, appelé également SR144528) sont fournis par Sanofi-Aventis Recherche (Montpellier, France).

### 1.3. Culture cellulaire et traitement

Des mélanocytes épidermiques humains normaux (NHEM) isolés de prépuce et fournis par Promocell (Heidelberg, Allemagne), sont cultivés pendant 24h à 37°C dans une atmosphère humide à 5% de CO₂ dans un milieu de culture adapté M2 (Promocell).

L'AEA et les autres composés sont ajoutés directement dans le milieu de culture. 24h après chaque traitement, la viabilité des cellules a été mesurée par l'utilisation du colorant d'exclusion bleu Trypan (Maccarone et al., 2003).

Le mAEA, l'AEA et l'ACEA sont testés *in vitro* à une concentration de 1 µM et comparés. Le mAEA est aussi utilisé à cette concentration quand il est utilisé en combinaison avec le SR1 à 0,1 µM.

Par ailleurs, le mAEA a aussi été testé, seul, à des concentrations de 0,5 ; 1.0 ; 1,5 ; 2.0; 2.5 et 3.0 µM.

### 1.4. Détermination du contenu en mélanine des mélanocytes

La quantité en mélanine présente dans les cellules NHEM est mesurée selon une méthode connue de l'homme du métier (Xiao et al., Arch Dermatol Res 2007 ; 299 : 245-57). Selon cette méthode, les cellules NHEM sont traitées à la trypsine et lavées deux fois avec un tampon phosphate salin. Ensuite les échantillons sont resuspendus dans 200 µl d'eau MilliQ et 1 ml d'un mélange éthanol : éther (1:1, vol/vol) est ajouté pour éliminer les substances opaques autres que la mélanine. Après 15 minutes, les échantillons sont centrifugés à 600g pendant 5 minutes. Le culot est séché à l'air et dissout dans 200 µl de soude 1N. Les échantillons sont ensuite chauffés à 80°C pendant une heure et refroidis.

La quantité de mélanine est déterminée par spectrophotométrie à 450 nm en utilisant les contrôles standards de mélanine fournis par Sigma Chemical Co., de façon à vérifier la gamme de linéarité de l'essai. La quantité de mélanine est exprimée par rapport au contrôle.

### 1.5. Analyse statistique

Les résultats donnés sont les moyennes avec leurs écarts type de 3 essais indépendants, chacun réalisé en triplicata. Ils ont été comparés par analyse de variance (ANOVA).

### 2. Résultats

### 2.1. Démonstration de l'effet propigmentant des composés testés

| | **Contrôle** | **mAEA***** | **AEA***** | **ACEA***** | **mAEA+5R1^{###}** | **mAEA+SR2***** |
|---|---|---|---|---|---|---|
| **Moyenne** | 1,10 | 2,80 | 2,09 | 2,14 | 1,22 | 2,34 |
| **Ecart-type (SD)** | 0,10 | 0,26 | 0,22 | 0,21 | 0,09 | 0,30 |
| **Erreur Standard (SEM)** | 0,06 | 0,15 | 0,13 | 0,12 | 0,05 | 0,17 |

| | | | | | | |
|---|---|---|---|---|---|---|
| ***p<0.001 vs contrôle ^{###} p<0.001 *vs* mAEA | | | | | | |

L'anandamide et ses deux analogues, mAEA et ACEA, augmentent de manière significative la quantité de mélanine dans les mélanocytes (NHEM) par l'intermédiaire du récepteur CB1. Le mécanisme d'action par l'intermédiaire du récepteur CB1 est mis en évidence au vu des résultats obtenus par l'utilisation du SR1 en association avec le methanandamide : on observe en effet qu'un antagoniste spécifique du récepteur CB1, comme le SR1, empêche le mAEA d'avoir son effet pro-pigmentant. Le SR2, antagoniste spécifique du récepteur CB2, n'empêche par contre pas le mAEA d'avoir cet effet.

### 2.2. Démonstration de la relation effet / dose pour le mAEA

| | **Contrôle** | **0,5** | **1***** | **1,5***** | **2***** | **2**,**5***** | **3***** |
|---|---|---|---|---|---|---|---|
| **Moyenne** | 1,02 | 1,75 | 2,80 | 3,30 | 3,34 | 3,75 | 4,13 |
| **Ecart-type (SD)** | 0,08 | 0,20 | 0,26 | 0,26 | 0,50 | 0,19 | 0,41 |
| **Erreur standard (SEM)** | 0,04 | 0,12 | 0,15 | 0,15 | 0,29 | 0,11 | 0,23 |

| | | | | | | | |
|---|---|---|---|---|---|---|---|
| ***p<0.001 vs contrôle | | | | | | | |

Il a été démontré clairement que le mAEA induit une augmentation dose-dépendante de la production de mélanine dans les cellules NHEM.

### 2.3. Conclusions

Compte tenu de ces résultats, l'anandamide et ses deux analogues cités ci-dessus peuvent donc être utilisés efficacement pour favoriser la coloration de la peau et/ou des poils et/ou des cheveux. En augmentant les teneurs en mélanine dans la peau ou dans les cheveux, ces composés miment donc la coloration naturelle de ces matières kératiniques.

### Exemple 2: Compositions cosmétiques

### Crème pro-pigmentante

| **Ingrédients** | **Quantités (en gramme)** |
|---|---|
| Anandamide | 5,00 |
| Ceteareth 30 | 7,00 |
| Stéarate de glycéryle | 2,00 |
| Alcool cétylique | 1,50 |
| Polydiméthylsiloxane | 1,50 |
| Huile de paraffine | 15,00 |
| Glycérine | 20,00 |
| Conservateurs | q.s. |
| Eau déminéralisée | qsp 100,00 g |

Après application de cette crème sur la peau, on observe une augmentation de la coloration de la peau.

### Lotion pour les cheveux

| **Ingrédients** | **Quantités (en gramme)** |
|---|---|
| Methanandamide | 0,50 |
| Propylène glycol | 30,00 |
| Alcool éthylique | 40,50 |
| Eau | qsp |

Après application de cette lotion sur une chevelure ayant des cheveux blancs, on observe une diminution du blanchissement naturel des cheveux.

### Exemple 3 Evaluation de l'effet propigmentant de mAEA sur epidermes reconstruits pigmentés (ERP)

Des épidermes pigmentés sont reconstruits sur un support collagénique (BPER) à l'aide de mélanocytes et kératinocytes humains normaux.
Les épidermes sont obtenus après 8 jours de reconstruction, les mélanocytes choisis sont de phototype estimé IV.1
Certains épidermes sont traités quotidiennement par le placebo, d'autres par le produit à tester mAEA à différentes concentrations (1µM et 3 µM).

Chaque condition expérimentale est réalisée en quadruplicat.

La qualité histologique des épidermes après traitement est évaluée sur coupes histologiques après coloration HES afin de déterminer l'impact du traitement sur les épidermes. Aussi la physiologie des mélanocytes est étudiée pour détecter une éventuelle mélano-cytotoxicité. Si aucune altération histologique ni aucun effet mélano-toxique du produit évalué n'est révélé, une quantification de la pigmentation est effectuée par quantification de la mélanine sur coupes histologiques par analyse d'images

### Analyse des résultats

Une quantification de la pigmentation par quantification microscopique de la mélanine est alors réalisée et permet d'objectiver une activité propigmentante du composé à tester.

La mélanine présente dans l'épiderme est colorée sur coupes histologiques (coloration Fontana Masson) puis quantifiée par analyse d'images. Pour cela, chaque épiderme est photographié sur toute sa largeur à l'aide d'un microscope. Environ 10 images sont acquises par épiderme (lumière blanche, grossissement X20).
La surface occupée par la mélanine est quantifiée à l'aide d'un logiciel d'analyse d'image développé à façon. Brièvement, chaque image est segmentée en 4 zones selon leur couleur (lumière, stratum corneum, épiderme vivant et BPER). La mélanine présente dans l'épiderme sous forme de grains noirs est seuillée comme étant la zone la plus sombre de l'épiderme sur le plan rouge. Les pixels occupés par la mélanine dans l'épiderme entier ou les couches vivantes sont ensuite quantifiés.
Cette quantification rend compte de la surface occupée par la mélanine ainsi que de la localisation moyenne de la mélanine dans l'épiderme.

Les résultats ci-dessous sont exprimés en pourcentage de stimulation relative par rapport au témoin cultivé en milieu de co-culture standard.

**Tableau 1**

| composé testé | % | valeur |
|---|---|---|
| Ethanol SYST D17 | 100 | 12,5 |
| Methanandamide 1µM SYST D17 | 315 | 25 |
| Methanandamide 3µM SYST D17 | 356,25 | 37,5 |

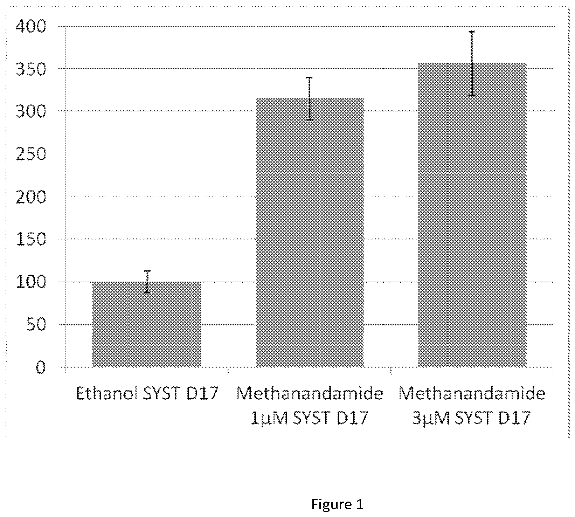

Les résultats sont présentés dans la figure 1.

On observe que le mAEA induit une augmentation très importante de la production de mélanine dans les épidermes pigmentés reconstruits, de manière dose-dépendante.

### Exemple 4 : Evaluation de l'effet propigmentant sur NHEM ou NHEM-NHEK

Des mélanocytes humains primaires (NHEM) on été ensemencés en plaques 24-puits en présence (co-culture) de kératinocytes épidermiques humains normaux (NHEK) puis cultivés pendant 24 h. Les cellules ont ensuite été traitées avec rien (témoin négatif), IBMX (témoin positif, 200 µM), ou méthanandamide (mAEA, 1 µM).
Après traitement, les cellules on été incubées pendant 72 h, toutes les conditions expérimentales ayant été réalisées en triplicat.

Après 72 h d'incubation, les cellules ont été lysées par une solution de NaOH 0,5 N afin d'extraire la mélanine.

Une quantification de la pigmentation par quantification microscopique de la mélanine est alors réalisée et permet d'objectiver une activité propigmentante du composé à tester.

La densité optique des échantillons a été mesurée à 405 nm, en référence à une gamme de mélanine exogène (courbe de mélanine standard 0,39 à 100 µg/ml).

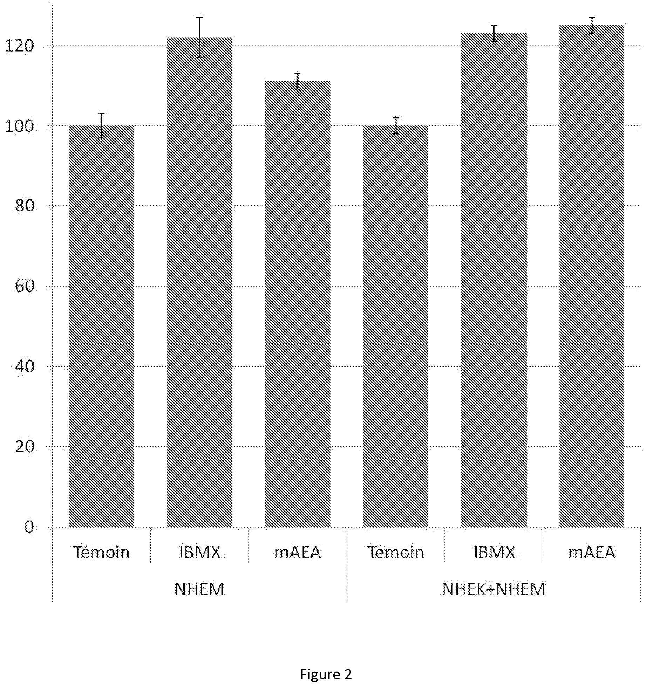

Les résultats sont présentés dans la figure 2.

On observe que le mAEA induit une augmentation de la production de mélanine dans les cellules NHEM ainsi que dans les cellules NHEK+NHEM. La production de mélanine dans NHEK+NHEM est plus importante que dans NHEM seul, dépassant même celle du témoin positif IBMX.

## Revendications

1. Utilisation cosmétique non-thérapeutique d'au moins un composé cannabinoïde choisi parmi l'anandamide, le methanandamide, l'arachidonoyl 2'-chloroethylamide, le linoleyl ethanolamide, le docosatetraenyl ethanolamide et le 2-arachidonyl glyceryl ether,
comme agent de coloration des matières kératiniques.

2. Utilisation selon la revendication 1, comme agent pour augmenter la pigmentation de la peau et/ou des poils et/ou des cheveux.

3. Utilisation selon l'une quelconque des revendications 1 ou 2, comme agent de brunissement de la peau.

4. Utilisation selon l'une quelconque des revendications 1 ou 2, comme agent pour ralentir le blanchissement naturel des cheveux.

5. Utilisation selon l'une quelconque des revendications précédentes, comme agent pour stimuler la mélanogénèse.

6. Procédé cosmétique non-thérapeutique de coloration de la peau comprenant l'application topique sur la peau ou l'administration orale, d'une composition comprenant, dans un milieu physiologiquement acceptable, un composé cannabinoïde tel que défini dans la revendication 1.

7. Procédé cosmétique non-thérapeutique de traitement du blanchissement naturel des cheveux comprenant l'application topique sur les cheveux ou l'administration orale, d'une composition comprenant, dans un milieu physiologiquement acceptable, un composé cannabinoïde tel que défini dans la revendication 1.

8. Procédé selon l'une quelconque des revendications 6 ou 7, **caractérisé en ce que** ledit composé cannabinoïde est présent à une concentration comprise entre à 0,00001 et 10% en poids par rapport au poids total de la composition.

## Claims

1. Non-therapeutic cosmetic use of at least one cannabinoid compound chosen from anandamide, methanandamide, arachidonyl-2'-chloroethylamide, linoleyl ethanolamide, docosatetraenyl ethanolamide and 2-arachidonyl glyceryl ether,
as an agent for colouring keratin materials.

2. Use according to Claim 1, as an agent for increasing the pigmentation of the skin and/or body hair and/or head hair.

3. Use according to either one of Claims 1 and 2, as a skin-browning agent.

4. Use according to either one of Claims 1 and 2, as an agent for slowing down the natural whitening of head hair.

5. Use according to any one of the preceding claims, as an agent for stimulating melanogenesis.

6. Non-therapeutic cosmetic process for colouring the skin, comprising the topical application to the skin, or the oral administration, of a composition comprising, in a physiologically acceptable medium, a cannabinoid compound as defined in Claim 1.

7. Non-therapeutic cosmetic process for treating the natural whitening of head hair, comprising the topical application to head hair, or the oral administration, of a composition comprising, in a physiologically acceptable medium, a cannabinoid compound as defined in Claim 1.

8. Process according to either one of Claims 6 and 7, **characterized in that** said cannabinoid compound is present at a concentration of between 0.00001% and 10% by weight relative to the total weight of the composition.

## Patentansprüche

1. Nichttherapeutische kosmetische Verwendung von mindestens einer Cannabinoidverbindung ausgewählt aus Anandamid, Methanandamid, Arachidonoyl-2'-chlorethylamid, Linoleylethanolamid, Docosatetraenylethanolamid und 2-Arachidonylglycerylether als Färbemittel für Keratinsubstanzen.

2. Verwendung nach Anspruch 1 als Mittel zum Erhöhen der Pigmentierung der Haut und/oder der Körperhaare und/oder des Kopfhaars.

3. Verwendung nach einem der Ansprüche 1 oder 2 als Bräunungsmittel für die Haut.

4. Verwendung nach einem der Ansprüche 1 oder 2 als Mittel zum Verlangsamen des natürlichen Weißwerdens des Kopfhaars.

5. Verwendung nach einem der vorhergehenden Ansprüche als Mittel zum Stimulieren der Melanogenese.

6. Nichttherapeutisches kosmetisches Verfahren zum Färben der Haut, umfassend die topische Applikation auf die Haut oder die orale Verabreichung einer Zusammensetzung, die eine Cannabinoidverbindung wie in Anspruch 1 definiert in einem physiologisch unbedenklichen Medium umfasst.

7. Nichttherapeutisches kosmetisches Verfahren zum Behandeln des natürlichen Weißwerdens des Kopfhaars, umfassend die topische Applikation auf das Kopfhaar oder die orale Verabreichung einer Zusammensetzung, die eine Cannabinoidverbindung wie in Anspruch 1 definiert in einem physiologisch unbedenklichen Medium umfasst.

8. Verfahren nach einem der Ansprüche 6 oder 7, **dadurch gekennzeichnet, dass** die Cannabinoidverbindung in einer Konzentration zwischen 0,00001 und 10 Gew.-% in Bezug auf das Gesamtgewicht der Zusammensetzung vorliegt.
